# EUROPEAN PATENT APPLICATION

(11) **EP 1 857 454 A1**
(43) Date of publication of application: **21.11.2007**
(21) Application number: 07008292.0
(22) Date of filing: 24.04.2007
(51) Int. Cl.: C07D 401/04

(54) **Crystalline imatinib base and production process therefor**

(30) Priority: 15.05.2006 US 433941
(71) Applicant: CHEMAGIS LTD., 51200 Bnei Brak (IL)
(72) Inventor: Adin, Itai, Beer Sheva 84684 (IL); Futerman, Yuri, Beer Sheva 84000 (IL); Iustain, Carmen, Beer Sheva 84750 (IL)
(74) Representative: Albrecht, Thomas

(57) **Abstract**

Provided is crystalline imatinib base form I and processes for producing crystalline imatinib base form I, which is suitable for preparing imatinib salts such as, e.g., the mesylate salt. Also provided is a process for producing a salt of imatinib from crystalline imatinib base form I.

## Description

### BACKGROUND OF THE INVENTION

Imatinib (N-{5-[4-(4-methyl-piperazinomethyl)-benzoylamido]-2-methylphenyl}-4- (3-pyridyl)-2-pyrimidine-amine) is represented by the following structural formula (I):

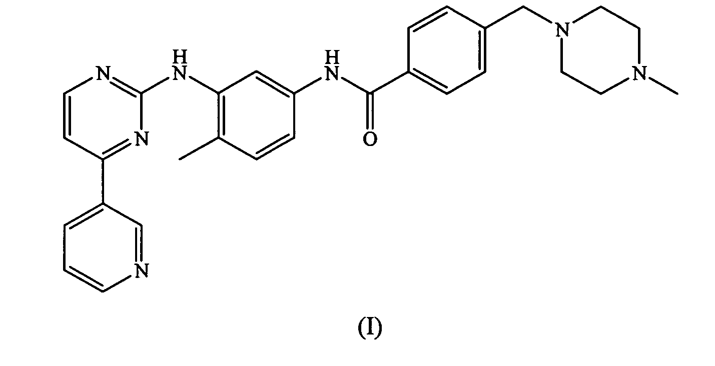

Imatinib is known as an inhibitor of tyrosine kinases and is indicated for the treatment of chronic myeloid leukemia (CML), Philadelphia chromosome positive leukemia, for patients in chronic phase and in blast crisis, accelerated phase and also for malignant gastrointestinal stromal tumors. It selectively inhibits activation of target proteins involved in cellular proliferation. Imatinib also has potential for the treatment of other cancers that express these kinases, including acute lymphocytic leukemia and certain solid tumors. Imatinib is sold by Novartis under the name Gleevec™ capsules, which contain imatinib mesylate equivalent to 100 mg of imatinib free base.

The preparation of imatinib base is described in example 21 of EP 0 564 409; however, the solid state properties of the base are not characterized. U.S. Patent No. 6,894,051 describes two crystalline forms of imatinib mesylate, the α-form and the β-form, and WO 2004/106326 describes a crystalline form of imatinib mesylate, designated as form H1, an amorphous imatinib mesylate hydrate and crystalline imatinib mesylate hydrate. WO 2005/095379 and WO 2006/024863 describe methods of preparing the imatinib mesylate α- form.

WO 2005/075454 describes acid addition salts of imatinib such as imatinib tartrate, citrate, malate, fumarate, etc., which are prepared by treatment of imatinib base with the corresponding acid. Imatinib base is the precursor of salt forms of imatinib. As such, there is a need for a stable, crystalline imatinib base polymorph, which may be conveniently used as the precursor in the preparation of imatinib salts, e.g., the mesylate salt, the tartrate salt, etc., and a process for preparing such a polymorph. The present invention provides such a polymorph and process.

### SUMMARY OF THE INVENTION

The present invention provides crystalline imatinib base form I. The present invention further provides a process for preparing crystalline imatinib base form I using a crystallization process. The imatinib base starting material can be produced by any suitable method, including synthesis methods known in the art. Preferably, the imatinib base is obtained as described in our U.S. Patent Application No. 11/318,455, entitled "Novel process for preparing imatinib," filed on December 28, 2005, which is incorporated by reference as if fully set forth herein. U.S. Patent Application No. 11/318,455 describes a novel process for producing imatinib base, which avoids the use of the highly toxic and corrosive reagent cyanamide. See also Reference Example 1 herein.

The process of the present invention preferably includes dissolving imatinib base, which can be obtained as per any suitable method, e.g., as per Reference Example 1 herein and methods known in the art, in a solvent and heating; allowing the solution to cool sufficiently to produce crystals of imatinib base form I; isolating the crystals, e.g., by filtration and, optionally, drying the crystals. The process of the present invention produces high purity crystalline imatinib base form I, which can be used as a convenient precursor for preparing imatinib salts, e.g., imatinib mesylate. Imatinib base crystalline form I produces a unique X-ray powder diffraction pattern as depicted in Fig. 1 and Table 1, a unique Infra Red spectrum, which is depicted in Fig. 2 and a DSC curve, which is depicted in Fig. 3.

The present invention further provides a process for preparing a salt of imatinib from crystalline imatinib base form I. The process preferably includes converting crystalline imatinib base form I into a salt of imatinib. In one embodiment, the process includes reacting imatinib base with an acid to produce an acid addition salt of imatinib. An exemplary process includes reacting imatinib base with methanesulfonic acid to produce imatinib mesylate.

### BRIEF DESCRIPTION OF THE DRAWINGS

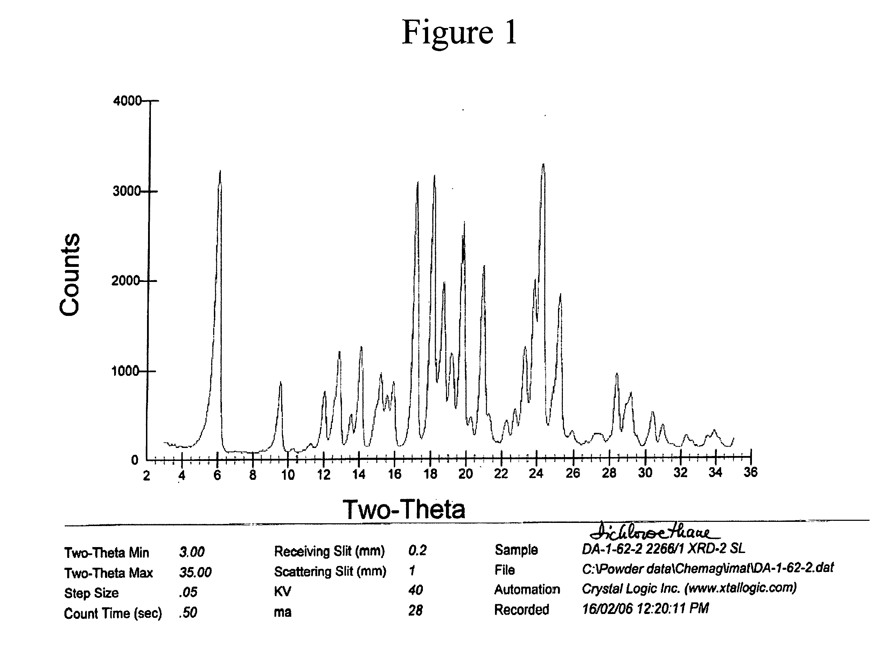

Figure 1 depicts the X-ray powder diffraction pattern of imatinib base crystalline form I.

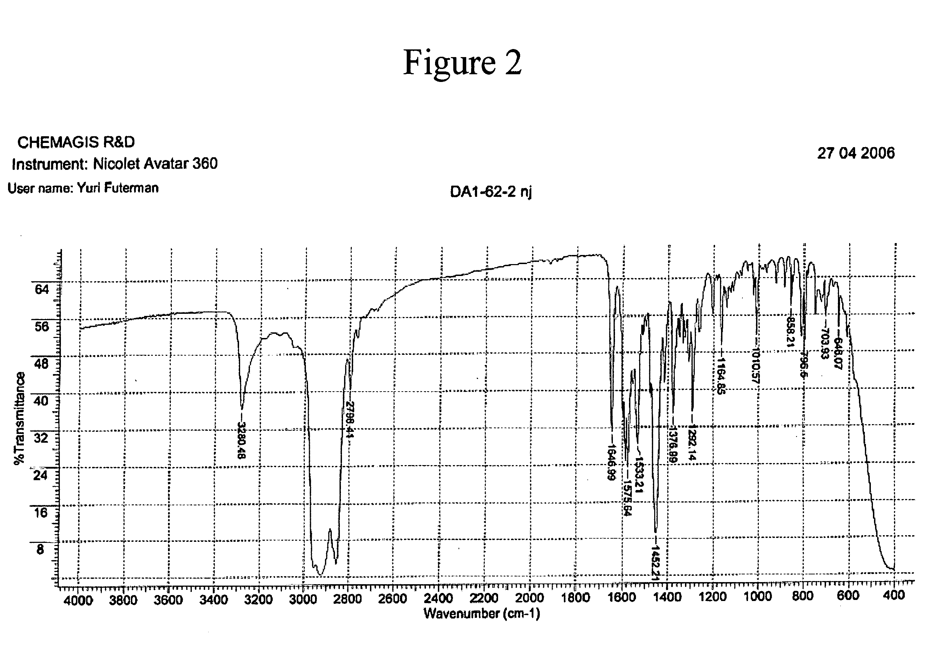

Figure 2 depicts the Infra-Red spectrum of imatinib base crystalline form I.

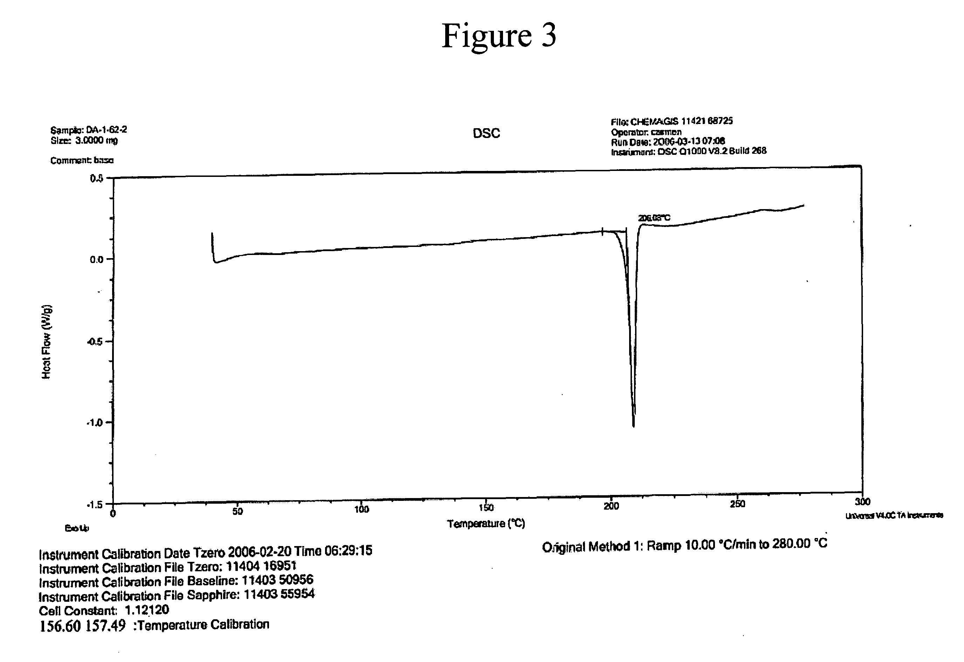

Figure 3 depicts the DSC curve of imatinib base crystalline form I.

### DETAILED DESCRIPTION OF THE INVENTION

Imatinib base crystalline form I produces a unique X-ray powder diffraction pattern as depicted in Fig. 1 and Table 1. The strong diffraction peaks at 6.0, 17.2, 18.1, 18.7, 19.8, 20.9, 23.8, 24.3, and 25.2±0.2 degrees 2θ are most characteristic of this form. The X-ray powder diffraction peak position and intensities exhibited by imatinib base crystalline form I are listed in Table 1.

**Table 1**

| Peak position 2θ degrees | Relative intensity I/I₀ | | Peak position 2θ degrees | Relative intensity I/I₀ |
|---|---|---|---|---|
| 6.0 | 100.0 | | 21.3 | 4.7 |
| 9.6 | 14.8 | | 22.3 | 4.3 |
| 11.3 | 1.5 | | 22.7 | 6.6 |
| 12.0 | 12.1 | | 23.3 | 18.4 |
| 12.8 | 19.1 | | 23.8 | 33.3 |
| 13.5 | 7.7 | | 24.3 | 81.7 |
| 14.1 | 21.6 | | 25.2 | 29.9 |
| 15.2 | 14.4 | | 25.9 | 2.6 |
| 15.6 | 10.0 | | 27.3 | 1.5 |
| 15.9 | 13.2 | | 28.4 | 15.0 |
| 17.2 | 52.1 | | 29.2 | 10.7 |
| 18.1 | 53.2 | | 30.4 | 6.7 |
| 18.7 | 32.6 | | 31.0 | 4.6 |
| 19.1 | 17.3 | | 32.3 | 2.8 |
| 19.8 | 45.1 | | 33.5 | 2.5 |
| 20.3 | 5.2 | | 33.9 | 3.8 |
| 20.9 | 37.6 | | | |

In addition, imatinib base crystalline form I produces a unique Infra-Red spectrum as depicted in Fig. 2. Further, imatinib base crystalline form I produces a characteristic DSC curve as depicted in Fig. 3.

In one embodiment, the present invention provides a process for preparing crystalline imatinib base form I, which includes crystallizing imatinib base, obtained, e.g., as per Reference Example 1 herein, from different solvents. An exemplary process for preparing imatinib base crystalline form I in accordance with the present invention includes the steps of:
dissolving imatinib base, obtained as per any suitable method including the method described per Reference Example 1 and methods known in the art, in a solvent and heating;
allowing the solution to cool sufficiently, e.g., to produce crystals of imatinib base form I; and
isolating the crystals (e.g., collecting the crystals by filtration) and, optionally, drying the crystals.

Preferably, the solvent used to obtain the imatinib base crystalline form I in accordance with the present invention includes toluene, chloroform, dichloroethane, methyl ethyl ketone (MEK), methyl isobutyl ketone (MIBK), cyclohexanone, 4-methylcyclohexanone, ethyl acetate, methanol, isopropyl alcohol, n-butanol and mixtures thereof.

The process of the present invention preferably produces crystalline imatinib base form I, e.g., when crystallized from n-butanol or from ethyl acetate, in at least about 80% yield, and more preferably in at least about 92% yield.

The process of the present invention preferably produces crystalline imatinib base form I in a purity of at least about 98.8%, and more preferably in a purity of at least about 99.5%.

The ratio between the imatinib base starting material and the crystallization solvent (imatinib base:solvent ratio) preferably is at least about 1 mmol imatinib base:6ml solvent (1:6 mmol imatinib base:ml solvent).

Inasmuch as imatinib base is reportedly heat sensitive, the processes for preparing crystalline imatinib form I of the present invention is preferably carried out under nitrogen and at a temperature which will not cause unwanted degradation of imatinib base. In one embodiment, the process of the present invention is carried out at a temperature of at least about 84°C but below the temperature at which significant degradation of the imatinib occurs.

The present invention further provides a process for preparing a salt of imatinib from crystalline imatinib base form I. The process preferably includes converting crystalline imatinib base form I into a salt of imatinib. In one embodiment, the process includes reacting imatinib base (obtained, e.g., by dissolving at least a portion of crystalline imatinib base form I in a suitable solvent) with an acid to produce an acid addition salt of imatinib. An exemplary process includes reacting imatinib base with methanesulfonic acid to produce imatinib mesylate.

### EXAMPLES

The following examples further illustrate the invention but, of course, should not be construed as in any way limiting its scope.

General description of the equipment:
X-ray diffraction data were acquired using a PHILIPS X-ray diffractometer model PW1050-70. System description: K_{α1}=1.54178Å, voltage 40kV, current 28 mA, diversion slit=1°, receiving slit=0.2mm, scattering slit=1° with a Graphite monochromator. Measurements of 2θ values typically are accurate to within ±0.2 degrees. Experiment parameters: pattern measured between 2θ=3° and 2θ=30° with 0.05° increments; count time was 0.5 second per increment.

Infra-red spectra were run on Nicolet Fourier-transform infra-red spectrometer model Avatar 360, with Omnic software version 5.2. All samples were run as KBr disks. The current infra-red measurements are accurate to within 4 cm⁻¹.

Differential scanning calorimetry (DSC) measurements were run on TA instruments model Q1000, with Universal software version 3.88. Samples were analyzed inside crimped 40 µl Aluminum pans. Heating rate for all samples was 10 °C/min.

### REFERENCE EXAMPLE 1

A mixture of 8.6g (0.031 mol) N-(5-amino-2-methylphenyl)-4-(3-pyridyl)-2-pyrimidine-amine and 12.6g (0.038 mol) of 4-(4-methyl-piperazinomethyl)-benzoyl chloride in pyridine (350 ml) were stirred under nitrogen at 50°C for 4.5 hours. The solvent was evaporated under reduced pressure at a temperature of about 70°C. Water (350 ml) was added, and the solution was basified to pH 10 with 6N NaOH (about 20 ml). The aqueous solution was extracted with dichloromethane (2X250ml), and dried over Na₂SO₄. After the solvent was evaporated, ethyl acetate was added to the thus formed residue (80 ml), which was slurried and filtered. Another portion of ethyl acetate was added (40 ml) and the residue was slurried for one more time, filtered and dried to give N-{5-[4-(4-methyl-piperazinomethyl)-benzoylamido]- 2-methylphenyl}-4- (3-pyridyl)-2-pyrimidine-amine (imatinib base) in 70% yield, having a m.p of 210-213°C, and a purity of 97%, by HPLC.

### EXAMPLE 2

A three-necked reaction vessel equipped with a thermometer, a reflux condenser and a mixer was charged with 0.5 gram of imatinib base, which was obtained as per reference example 1 (1.01 mmoles) under nitrogen atmosphere and mixed with 20 ml of n-butanol. The mixture was heated to about 91°C until a clear solution was obtained. The solution was cooled to room temperature and the resulting crystals were washed with 2 ml of cold n-butanol, filtered and dried under reduced pressure to obtain 0.46 g of imatinib base form I in 92% yield. The purity was determined by HPLC (99.7%).

### EXAMPLE 3

A three-necked reaction vessel equipped with a thermometer, a reflux condenser and a mixer was charged with 0.5 gram of imatinib base (1.01 mmoles) under nitrogen atmosphere and mixed with an organic solvent. The list of the organic solvents that were used and the volume of each solvent are summarized in Table 2. The mixture was heated to a certain temperature, as specifically detailed in Table 2 for each solvent, until a clear solution was obtained. The solution was cooled to room temperature and the resulting crystals were filtered and dried under reduced pressure.

**Table 2 - Results with different solvents**

| Entry | Solvent | Volume, ml | Temperature, °C | Yield % | Purity, % |
|---|---|---|---|---|---|
| 1 | toluene | 110 | 84 | 60 | n.d |
| 2 | cyclohexanone | 6 | 84 | 80 | n.d |
| 3 | chloroform | 6 | reflux | 70 | n.d |
| 4 | dichloroethane | 12 | 81 | 80 | n.d |
| 5 | acetonitile | 130 | reflux | 56 | 99.6 |
| 6 | methanol | 13 | reflux | 70 | 99.7 |
| 7 | MEK | 40 | reflux | 80 | 99.6 |
| 8 | MIBK | 60 | 112 | 60 | 97.0 |
| 9 | isopropyl alcohol | 40 | reflux | 80 | 99.6 |
| 10 | ethyl acetate | 95 | reflux | 92% | 98.9 |

| | | | | | |
|---|---|---|---|---|---|
| (n.d=not determined) | | | | | |

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. Crystalline imatinib base form I **characterized by** an X-ray powder diffraction pattern exhibiting strong diffraction peaks at 6.0, 17.2, 18.1, 18.7, 19.8, 20.9, 23.8, 24.3, and 25.2 ± 0.2 degrees 2θ.

2. Crystalline imatinib base form I of claim 1 further **characterized by** an Infra-Red spectrum exhibiting characteristic absorption peaks at 3280, 1647, 1533, 1292, 1165, 1010, 926, 858, and 703 ± cm⁻¹.

3. The crystalline solid comprising imatinib base form I of claim 1 further **characterized by** DSC curve exhibiting peak onset at 206±10°C.

4. A process for preparing the crystalline imatinib base form I, the process comprising:
dissolving imatinib base in a solvent and heating;
allowing the solution to cool sufficiently to produce crystals of imatinib base form I;
isolating the crystals; and
optionally washing and drying the crystals.

5. The process of claim 4, wherein the solvent comprises toluene, chloroform, dichloromethane, methyl ethyl ketone (MEK), methyl isobutyl ketone (MIBK), cyclohexanone, 4-methylcyclohexanone, ethyl acetate, methanol, isopropyl alcohol, n-butanol or a mixture thereof.

6. The process of claim 5, wherein the crystalline imatinib base form I is obtained in a purity of at least about 98.8%.

7. The process of claim 5, wherein the crystalline imatinib base form I is obtained in a purity of at least about 99.5%.

8. The process of claim 5, wherein the solvent comprises n-butanol or ethyl acetate and the yield of crystalline imatinib base form I is at least about 80%.

9. The process of claim 8, wherein the yield of crystalline imatinib base form I is at least about 92%.

10. A process for preparing a salt of imatinib, the process comprising converting crystalline imatinib base form I into a salt of imatinib.

11. The process of claim 10, wherein imatinib base is reacted with an acid, to produce an acid addition salt of imatinib.

12. The process of claim 11, wherein the acid is methanesulfonic acid and the acid addition salt is imatinib mesylate.

13. Imatinib base having a purity, which is equal to or greater than 99.5%.
